# EUROPEAN PATENT SPECIFICATION

(11) **EP 2 747 561 B1**
(45) Date of publication and mention of the grant of the patent: **09.05.2018**
(21) Application number: 12825447.1
(22) Date of filing: 23.08.2012
(51) Int. Cl.: A61K 31/166, A61K 9/00, A61K 47/12, A61K 47/18, A61P 1/00, A61P 43/00

(54) **TREATMENT OF SYMPTOMS ASSOCIATED WITH FEMALE GASTROPARESIS**
BEHANDLUNG VON SYMPTOMEN IM ZUSAMMENHANG MIT WEIBLICHER GASTROPARESE
TRAITEMENT DE SYMPTÔMES ASSOCIÉS À LA GASTROPARÉSIE CHEZ LA FEMME

(30) Priority: 25.08.2011 US 201161527563 P; 05.01.2012 US 201261583447 P
(43) Date of publication of application: 02.07.2014
(73) Proprietor: Evoke Pharma, Inc., Solana Beach, CA 92075 (US)
(72) Inventor: D'ONOFRIO, Matthew, J., Solana Beach, CA 92075 (US); GONYER, David, A., Cardiff, CA 92009 (US); CARLSON, Marilyn, R., Encinitas, CA 92024 (US)
(74) Representative: Wichmann, Hendrik
(86) International application number: PCT/US2012/052096
(87) International publication number: WO 2013/028882

(56) References cited:
- WO-A1-01/74350
- WO-A2-2010/075444
- US-A1- 2002 065 321
- US-A1- 2005 137 265
- WO ET AL.: 'Motility and Functional Disorders of the Stomach: Diagnosis and Management of Functional Dyspepsia and Gastroparesis. Practical Gasteroenterology' GI MOTILITY, A SERIES FROM THE AMS, SERIES #4 2006, pages 23 - 48, XP008173114
- H. P. PARKMAN ET AL: "Metoclopramide nasal spray is effective in symptoms of gastroparesis in diabetics compared to conventional oral tablet", NEUROGASTROENTEROLOGY & MOTILITY, vol. 26, no. 4, 25 December 2013 (2013-12-25), pages 521-528, XP055152585, ISSN: 1350-1925, DOI: 10.1111/nmo.12296
- Anon: "Gastroparesis: Clinical Evaluation of Drugs for Treatment Guidance for Industry", , 1 July 2015 (2015-07-01), pages 1-10, XP055398900, Rockville, USA Retrieved from the Internet: URL:https://www.fda.gov/downloads/drugs/gu idancecomplianceregulatoryinformation/guid ances/ucm455645.pdf [retrieved on 2017-08-16]

## Description

### BACKGROUND OF THE INVENTION

Metoclopramide is approved in the United States in oral solution, oral tablet, orally dissolving tablet and injectable solution forms. Wenig has suggested the use of nasally-administered metoclopramide for the treatment of emesis or nausea. (See United States Patent No. 4,624,965, issued November 25, 1986.) Psilogenis has suggested nasal administration of metoclopramide for the treatment of delayed onset emesis. (See United States Patent No. 5,760,086, issued June 2, 1998.) Lehman et al. have proposed administering nasal formulations of metoclopramide for the treatment of gastroparesis. (*See* United States Patent No. 6,770,262, issued August 3, 2004.) WO 2010/075444 discloses an outline for a clinical study for a nasal formulation of metoclopramide, but results are not provided. WO 01/74350 (and its family member US 2002/065321) describe a clinical report without placebo control examining the clinical efficacy of nasal metoclopramide for the treatment of symptoms of gastroparesis, but females are not identified as a sub-group of patients. US 2005/137265 described oral dosage forms of metoclopramide for the treatment of the symptoms of gastroparesis.

### SUMMARY OF THE INVENTION

At the direction of the inventors, a clinical study of the efficacy of intranasal metoclopramide was carried out in male and female gastroparesis patients. As an outcome of the clinical study, the inventors discovered that, despite similarities in pharmacokinetics between women and men who received nasal metoclopramide, female gastroparesis responded positively to nasal metoclopramide compared to placebo, whereas male gastroparesis did not. No previous study has noted any difference in response compared to placebo by metoclopramide between female and male gastroparesis patients. Even taking into account known differences between females and males, such as differences in mean body weight and frequency of occurrence of gastroparesis in the two sexes, the difference in response to nasal metoclopramide was statistically significant. Thus the inventors have discovered that, at least at the doses administered in the study, nasal administration of metoclopramide is effective in the treatment of symptoms associated with female gastroparesis, but not in the treatment of symptoms associated with male gastroparesis.

Thus, some embodiments described herein relate to a formulation of metoclopramide as defined in the claims for intranasal administration to a diabetic human female, for use in the treatment of one or more symptoms associated with diabetic gastroparesis in human females. In some embodiments, the metoclopramide is administered at a daily dose of 20 mg to 100 mg (*e.g.* 40 mg to 80 mg) of metoclopramide base per day. The daily dose of metoclopramide is administered as 3-8 intranasal aliquots of 5 mg to 25 mg (*e.g.,* 10 mg to 20 mg) of metoclopramide base per aliquot. In some embodiments, the daily dose of metoclopramide is administered as 3-8 intranasal aliquots of 10 mg of metoclopramide base per aliquot. In some particular embodiments, the intranasal aliquots are roughly equal. In some embodiments, each intranasal aliquot has a volume of 25 µL to 150 µL. In some embodiments, each intranasal aliquot has a volume of 50 µL. In some embodiments, the daily dose of metoclopramide is administered as 3-8 aliquots of about 14 mg metoclopramide base per aliquot. In some embodiments, each aliquot has a volume of 25 µL to 150 µL. In some embodiments, each aliquot has a volume of 70 µL. In some embodiments, the daily dose of metoclopramide is administered as 3 or 4 intranasal aliquots of 20 mg of metoclopramide base per aliquot. In some particular embodiments, the intranasal aliquots are roughly equal. In some embodiments, each intranasal aliquot has a volume of 50 µL to 150 µL. In some embodiments, the treatment of symptoms associated with female gastroparesis includes treatment of symptoms associated with female diabetic gastroparesis.

Some embodiments described herein provide a formulation as defined in the claims for use in the treatment of upper abdominal pain associated with diabetic gastroparesis in a human female. Some embodiments described herein provide a formulation as defined in the claims for use in the treatment of nausea associated with diabetic gastroparesis in a human female. Some embodiments described herein provide a formulation as defined in the claims for use in the treatment of bloating associated with diabetic gastroparesis in a human female. Some embodiments described herein provide a formulation as defined in the claims for use in the treatment of early satiety associated with diabetic gastroparesis in a human female. Some embodiments described herein provide a formulation as defined in the claims for use in the treatment of vomiting associated with diabetic gastroparesis in a human female. Some embodiments described herein provide a formulation as defined in the claims for use in the treatment of retching associated with diabetic gastroparesis in a human female. Some embodiments described herein provide a formulation as defoined in the claims for use in the treatment of feeling full (inability to finish a meal) associated with diabetic gastroparesis in a human female. Some embodiments described herein provide a formulation as defined in the claims for use in the treatment of loss of appetite associated with diabetic gastroparesis in a human female. Some embodiments described herein provide a formulation for use in the treatment of stomach fullness associated with diabetic gastroparesis in a human female. Some embodiments described herein provide a formulation as defined in the claims for use in the treatment of stomach being visibly larger associated with diabetic gastroparesis in a human female. Some embodiments described herein provide a formulation as defeined in the claims for use in the treatment of upper abdominal discomfort associated with diabetic gastroparesis in a human female subject. Some embodiments described herein provide a formulation as defined in the claims for use in the treatment of two, three, four, five, six or more symptoms associated with female diabetic gastroparesis selected from upper abdominal pain, nausea, bloating, early satiety, vomiting, retching, feeling full (inability to finish a meal), loss of appetite, stomach fullness, stomach being visibly larger, and abdominal discomfort. The administration of metoclopramide is intranasal administration of metoclopramide to a human female, as defined in the claims. Some embodiments provide for use of a composition described herein for the preparation of a medicament for the treatment of female diabetic gastroparesis.

Additional embodiments, features and advantages will become apparent upon consideration of the following detailed description of the invention.

### Brief Description of the Drawings

Figure 1 is a graph showing post-dose individual, arithmetic mean and geometric mean pharmacokinetic data (blood plasma levels in ng/mL) on visits 3 and 7 during a clinical study for all patients receiving intranasal (IN) metoclopramide at 10 mg per dose and 14 mg per dose.
Figure 2 is a graph showing post-dose individual, arithmetic mean and geometric mean pharmacokinetic data (blood plasma levels in ng/mL) on visits 3 and 7 of a clinical study for male and female patients receiving intranasal (IN) metoclopramide at 10 mg per dose and 14 mg per dose. No statistical difference between males and females was seen in the pharmacokinetic (PK) data.
Figure 3 is a graph showing mean mGCSI-DD total scores at baseline and change from baseline to week 4 in female subjects.
Figure 4 is a graph showing mean mGCSI-DD total scores at baseline and change from baseline to week 4 in male subjects.

### DETAILED DESCRIPTION OF THE INVENTION

The inventors have discovered that, despite similarities in pharmacokinetics and demographics between women and men who received nasal metoclopramide, intranasal administration of metoclopramide relieved symptoms associated with female gastroparesis, but not symptoms associated with male gastroparesis. Even taking into account known differences between females and males, such as differences in mean body weight and frequency of occurrence of gastroparesis in the two sexes, the difference in response to nasal metoclopramide was statistically significant. Thus the inventors have found that, at least at the doses administered in the clinical study, nasal administration of metoclopramide is effective in the treatment of symptoms associated with female gastroparesis, but not in the treatment of symptoms associated with male gastroparesis. Moreover, it is the belief of the inventors that, given the similar pharmacokinetics evinced by female and male humans in the intranasal studies, these results may be generalized to treatment of gastroparesis, and in particular diabetic gastroparesis.

Thus, some embodiments described herein relate to a formulation of metoclopramide as defined in the claims for intranasal administration for use in the treatment of symptoms associated with female diabetic gastroparesis, as further defined in the claims.

Thus, some embodiments described herein relate to a formulation as defined in the claims for use in treating at least one, preferably two or more, symptoms of female diabetic gastroparesis, as further defined in the claims. The administration of metoclopramide is intranasal. Some embodiments provided herein relate to a formulation as defined in the claims for use in treating at least one, preferably two or more, symptoms of female diabetic gastroparesis selected from the group consisting of: nausea (feeling sick to your stomach as if you were going to vomit or throw up); retching (heaving as if to vomit, but nothing comes up); vomiting; stomach fullness; not able to finish a normal-sized meal; feeling excessively full after meals; loss of appetite; bloating; stomach or belly visibly larger; and upper abdominal pain (above the navel); upper abdominal discomfort (above the navel). Some embodiments relate to a formulation as defined in the claims for use in treating two, three, four, five, six, seven, eight, nine, ten or all eleven of the symptoms selected from the group consisting of: nausea (feeling sick to your stomach as if you were going to vomit or throw up); retching (heaving as if to vomit, but nothing comes up); vomiting; stomach fullness; not able to finish a normal-sized meal; feeling excessively full after meals; loss of appetite; bloating; stomach or belly visibly larger; upper abdominal pain (above the navel); and upper abdominal discomfort (above the navel). The female gastroparesis is female diabetic gastroparesis.

As used herein, the term "female gastroparesis" refers to symptoms associated with gastroparesis experienced by human females.

As used herein "metoclopramide" refers to metoclopramide in a solution formulation, including a salt of metoclopramide. In quantitating the mass of metoclopramide herein, unless otherwise specified, all masses of metoclopramide refer to the mass of the free base, which has a molecular weight of 299.80. One method of manufacturing metoclopramide is described in US 3,177,252.

An "effective amount" of metoclopramide (or a pharmaceutically acceptable salt thereof) is an amount of metoclopramide that is effective to provide statistically significant relief from one or more symptoms of gastroparesis in a cohort of human females. An "effective amount" is determined in comparison to administration of placebo. In some embodiments, efficacy is judged with reference to the Gastroparesis Cardinal Symptom Index - Daily Diary (GCSI-DD) and in some embodiments, efficacy is judged with reference to the modified GCSI-DD (mGCSI-DD), which is described in more detail herein. An additional symptom measurement instrument is the Gastroparesis Symptom Assessment (GSA) may be used to measure efficacy. Although not specifically measured in the referenced study, the GSA is derived from, and has similar statistical outcomes to the mGCSI-DD. *See* Example 1.

As provided herein, an effective amount of metoclopramide for use in the treatment of symptoms associated with female diabetic gastroparesis, is ineffective to treat the symptoms associated with male gastroparesis. In some embodiments, the metoclopramide is administered at a daily dose of 20 mg to 60 mg of metoclopramide base per day. In some embodiments, the daily dose of metoclopramide is administered as 1 to 6 intranasal aliquots (*e.g*., sprays). In some embodiments, the daily dose of metoclopramide is administered as 4 intranasal aliquots. In some embodiments, the daily dose of metoclopramide is administered as 4 intranasal aliquots of 5 mg to 15 mg of metoclopramide base per aliquot. In some embodiments, the daily dose of metoclopramide is administered as 4 intranasal aliquots of 10 mg of metoclopramide base per aliquot. In some particular embodiments, the intranasal aliquots are roughly equal. In some embodiments, each intranasal aliquot has a volume of 25 µL to 150 µL. In some embodiments, each intranasal aliquot has a volume of 50 µL. In some embodiments, the daily dose of metoclopramide is administered as 4 aliquots of 14 mg metoclopramide base per aliquot. In some embodiments, each aliquot has a volume of 25 µL to 150 µL. In some embodiments, each aliquot has a volume of 70 µL.

Nasal metoclopramide for the treatment of female gastroparesis may be stable upon storage, especially long-term storage. The nasal metoclopramide solutions are, in some embodiments, clear and/or colorless. Some embodiments herein provide use of nasal metoclopramide formulations as defined in the claims for the preparation of a medicament for the treatment of symptoms associated with female diabetic gastroparesis.

There is provided a nasal metoclopramide formulation and its use in the treatment of symptoms associated with female diabetic gastroparesis, comprising metoclopramide (or a pharmaceutically-acceptable salt thereof), citrate buffer and benzalkonium chloride having a pH of at least about 5. In some embodiments, the nasal metoclopramide formulation is one described in copending United States patent application 12/645,108, filed December 22, 2009 (Published as US 2010/0163032 on July 1, 2010).

Also disclosed herein is a manufacture comprising a metoclopramide pharmaceutical composition, *e.g*. as described in copending United States patent application 12/645,108, filed December 22, 2009 (Published as US 2010/0163032 on July 1, 2010). In some embodiments, the means for nasal administration comprises a reservoir that contains the composition, a pump in fluid communication with the composition in the reservoir and a nozzle in fluid communication with the pump, wherein activation of the pump withdraws a predetermined amount of said composition from the reservoir and causes said predetermined amount of said composition to be expelled from said nozzle. In some embodiments, the predetermined amount of composition is about 10 µL to about 200 µL, about 10 µL to about 150 µL, about 50 µL to about 150 µL, about 50 µL, about 55 µL, about 60 µL, about 75 µL, about 70 µL, about 75 µL, about 80 µL, about 85 µL, about 90 µL, about 95 µL, about 100 µL, about 110 µL, about 120 µL, about 125 µL, about 150 µL, about 175 µL or about 200 µL per activation ("spray" or "aliquot"). In order to combat the deleterious effects of light on metoclopramide, the manufacture may conveniently include a container, especially an opaque container, *i.e.* a container that is at least partially or completely impervious to light. In some embodiments, a suitable opaque container will be brown or amber, especially brown or amber glass. In other embodiments, the opaque container will be an opaque polymer container, such as is commonly used in the pharmaceutical arts.

As used herein, the indefinite articles "a" and "an" mean "at least one" unless otherwise stated. Likewise, the definite article "the", unless otherwise indicated, means "at least the" where the context permits or demands it to be open-ended.

As used herein, a "nasal administration device" is a device capable of administering a dose of a composition comprising metoclopramide into the nose of a patient. In some embodiments, the nasal administration device is an atomizer, comprising a reservoir adapted to contain the metoclopramide solution and a pump adapted to draw a predetermined amount of the metoclopramide solution from the reservoir dispense the predetermined amount of metoclopramide solution through an atomizing nozzle and into at least one nostril of a patient. Suitable nasal administration devices are commercially available.

As used herein, the term "spray" indicates an atomized volume of liquid expelled from a nozzle of a nasal administration device upon a single activation of the nasal administration device. In general, each spray is administered into a single nostril of a patient. As such, a "spray", as used herein, is a type of "aliquot", the latter being a generic term referring to an amount of liquid sprayed, instilled or otherwise introduced into a nostril of a subject, such as a patient.

As used herein, "metoclopramide" means metoclopramide (-amino-5-chloro-N-(2-(diethylamino)ethyl)-2-methoxybenzamide) or a pharmaceutically acceptable salt thereof, such as the hydrochloride salt. Where reference is made to a particular mass of metoclopramide, the recited mass is that of the free base of metoclopramide, unless otherwise specified.

As used herein, "oral" means a dosage form taken by mouth, such as a tablet, powder, soft gel capsule, hard gel capsule, orally dissolving tablet or thin film, or liquid.

Other terms used herein have their art-recognized meanings, unless otherwise defined or described.

### Formulation of Nasal Compositions of Metoclopramide (Use for Production of a Medicament)

Nasal compositions of metoclopramide may be manufactured for administration as a medicament for administration to a patient for one of the indications described herein. In some embodiments, the nasal metoclopramide formulation is one described in copending United States patent application 12/645,108, filed December 22, 2009 (Published as US 2010/0163032 on July 1, 2010). Briefly, metoclopramide, buffer, benzalkonium chloride and optionally other ingredients (such as sodium chloride or other osmolarity-regulating agent, sorbitol or other sweetener, flavoring agent, etc.) may be made up to some volume less than the target final volume of the solution. The ingredients may then be mixed until all the ingredients are dissolved. The pH then may be adjusted, if necessary, by addition of a suitable acid or base, such as HCl, NaOH, or the complementary acid or base of the buffer. Once the desired pH has been obtained, the solution may then be brought up to full volume with water. The resulting solution may then be packaged in a suitable container for shipping and distribution. In some embodiments, the suitable container includes a nasal pump as described in more detail below. In other embodiments, the suitable container may be a vial, such as an amber glass vial, which may be a glass ampule, a glass bottle topped with an inert rubber septum and crimp cap top.

### Manufacture of Nasal Formulations

Some embodiments described herein provide, as a manufacture, a combination of a stable, clear and/or colorless solution of metoclopramide and a means for intranasal administration of the metoclopramide solution. In some embodiments, the manufacture comprises one of the metoclopramide solutions described herein and an intranasal delivery device comprising a reservoir, in which the metoclopramide solution is contained, a pump in fluid communication with the reservoir and a nozzle in fluid communication with the pump. In use, the pump is actuated, drawing an amount of the metoclopramide solution from the reservoir and expelling the solution out of the nozzle as an aerosolized spray. Suitable nasal administration devices are commercially available. Among the suppliers of nasal administration devices that may be combined with a stable, substantially clear and/or substantially colorless metoclopramide solution according to the present invention, there may be mentioned Aptar (Valois of America, Congers, New York, and Pfeiffer of America, Princeton, N.J.) In some embodiments, the intranasal delivery device is partially or completely opaque, in order to protect the contents of the device from exposure to ambient light.

### Therapeutic Uses Of Nasally Administered Metoclopramide

The nasal metoclopramide formulations described herein may be used for the treatment of symptoms associated with female diabetic gastroparesis, as further defeined in the claims.

In some embodiments provided herein, relief of symptoms associated with female gastroparesis is treated by intranasal instillation of a pharmaceutically effective amount of an intranasal metoclopramide solution, as defeined in the claims. In some embodiments, nasal metoclopramide is administered to female humans who have been diagnosed with diabetic gastroparesis. In some embodiments, an effective dose of nasal metoclopramide is administered to a female patient for about 1 to about 12 weeks, about 1 to 8 weeks, about 5 weeks to about 12 weeks, about 5 to about 8 weeks, or about 1, 2, 3, 4, 5, 6, 7, 8, 9, 10, 11, 12 or more weeks.

In some embodiments, the effective daily dose of metoclopramide is 20 mg/day to 100 mg/day, which may be administered in 1 to 8, 1 to 6, 1 to 4 or 1 to 3 aliquots (*e.g*. "sprays"). In some embodiments, the daily dose of metoclopramide is 40 mg/day to 80 mg/day. In some embodiments in which the patient is renally impaired or coadministered with a drug known to alter metabolism or clearance of metoclopramide, the dose may be decreased by 25-75%, *e.g.* to a daily dose of 20 mg, which may be administered in *e.g.* 4 aliquots of 5 mg each or 2 aliquots of 10 mg each. In some embodiments, the daily dose administered to women is effective in female gastroparesis but not male gastroparesis. In some embodiments, the daily dose of nasal metoclopramide is about 30 mg/day to about 80 mg/day, administered in 1, 2, 3, 4, 5, 6, 7 or 8 aliquots. In some embodiments, the daily dose is 20, 22, 24, 25, 26, 28, 30, 32, 34, 35, 36, 38, 40, 42, 44, 45, 46, 48, 50, 52, 54, 55, 56, 58, 60, 62, 64, 66, 68, 70, 72, 74, 76, 78 or 80 mg/day administered in 1, 2, 3, 4, 5 or 6 aliquots. In some embodiments, the aliquots are substantially equivalent in volume. In some embodiments, the volumes of the aliquots (*e.g*. "sprays") are 25 µL to 150 µL, *e.g.* 25 µL to 100 µL, 30 µL to 80 µL, 40 µL to 75 µL. In some embodiments, the volumes of the aliquots are 25-60 µL, 30-70 µL, 40-60 µL, 50-90 µL or 60-80 µL. In some embodiments, the volumes of the aliquots are 20 µL, 22 µL, 24 µL, 25 µL, 26 µL, 28 µL, 30 µL, 32 µL, 34 µL, 35 µL, 36 µL, 38 µL, 40 µL, 42, µL, 44 µL, 45 µL, 46 µL, 48 µL, 50 µL, 55 µL, 54 µL, 55 µL, 56 µL, 58 µL, 60 µL, 62 µL, 64 µL, 65 µL, 66 µL, 68 µL, 70 µL, 72 µL, 74 µL, 75 µL, 76 µL, 78 µL, 80 µL, 82 µL, 84 µL, 85 µL, 86 µL, 88 µL, 90 µL, 92 µL, 94 µL, 95 µL, 96 µL, 98 µL or 100 µL. In some embodiments, the total effective daily dose is 40 mg/day of metoclopramide base or 56 mg/day of metoclopramide base administered in four aliquots (4x50 µL or 4x70 µL) throughout the day. In some embodiments, the total effective daily dose is 80 mg/day of metoclopramide base administered in 8 aliquots (one in each nostril, four times throughout the course of the day.

In some embodiments, the therapeutic use comprises treatment of symptoms associated female gastroparesis associated with or caused by diabetes (including type 1 and type 2).

In some embodiments, the gastroparesis is of diabetic origin, including type 1 and type 2 diabetes, and treatment comprises intranasally administering a nasal composition of metoclopramide as described herein in a nasal spray dosage form for about 1 to about 8 weeks, for about 2 weeks to about 8 weeks or for 1, 2, 3, 4, 5, 6, 7, 8 or more weeks.

Administration may be prescribed 30 minutes before meals, assuming 3 meals per day, and before bedtime. In some embodiments, doses are administered before breakfast and dinner. In some embodiments, each dose is administered as a single intranasal aliquot (*e.g*. spray); in some embodiments, each dose is administered as 2 aliquots (*e.g*. one spray per nostril).

In some embodiments, a pharmaceutical composition administered for the treatment of symptoms associated with female diabetic gastroparesis as described herein consists of: metoclopramide (e.g. as metoclopramide HCl), citric acid (e.g. as the monohydrate), sodium citrate (e.g. as the dihydrate), benzalkonium chloride (e.g. as a 50% solution, N.F.), sorbitol (e.g. as a solution, such as a 70% solution USP), edetate disodium, sodium chloride and purified water. In some embodiments, a pharmaceutical composition administered for the treatment of female gastroparesis consists of: metoclopramide (e.g. as metoclopramide HCl), citric acid (e.g. as the monohydrate), sodium citrate (e.g. as the dihydrate), benzalkonium chloride (e.g. as a 50% solution, N.F.), edetate disodium, sodium chloride and purified water. In some embodiments, a pharmaceutical composition administered for the treatment of female diabetic gastroparesis consists of: metoclopramide (e.g. as metoclopramide HCl), citric acid (e.g. as the monohydrate), sodium citrate (e.g. as the dihydrate), benzalkonium chloride (e.g. as a 50% solution, N.F.), sodium chloride and purified water.

The nasal metoclopramide compositions described herein may be administered a female patient as 1 spray in a single nostril, four times a day (1 spray QID for about 1, 2, 3, 4, 5, 6, 7, or 8 weeks), or 1 spray per nostril in both nostrils four times a day (2 sprays QID for about 1, 2, 3, 4, 5, 6, 7, or 8 weeks).

In some embodiments, nasal metoclopramide is administered in the absence of other gastroparesis medications. In some embodiments, additional medication may be administered if necessary. In some embodiments, the therapeutic uses provided herein can also include co-administration of one or more additional therapeutic agents along with the metoclopramide nasal formulations described herein. The additional therapeutic agents administered concurrently with metoclopramide or at separate time intervals. In some embodiments, one or more other drugs may be incorporated into the metoclopramide nasal formulation. Additional therapeutic agents may include pain relievers, insulin and other drugs useful in the management of diabetes, steroids, especially steroids that prevent nasal irritation, and antidepressants.

Various techniques may be used to assess the severity of the gastroparesis and gastric emptying, and these will be well-known to those of skill in the art. Such techniques include questioning the patient regarding symptoms of gastroparesis by a Patient Reported Outcome (PRO) symptom measurement instrument. Techniques like octanoic breath test, wireless capsule endoscopy, radioscintigraphy, ultrasonography, and x-rays employing radiopaque markers such as barium, may be employed.

In some embodiments, a clinician will prescribe a lower dosage of metoclopramide because of an underlying medical condition or other clinical consideration. For example, in the case of renal impairment, the clinician will prescribe a dose that is appropriate for the degree of renal impairment or other rationale for slower metabolism or clearance of the metoclopramide, *e.g*. a dose that is 25% to 75% lower, in some embodiments 50% lower, than the dose prescribed for a patient without renal impairment. In some such embodiments, the daily dose will be 20 mg administered as two intranasal doses, *e.g.* one dose before breakfast and one before dinner. In some embodiments, each dose is administered as a single intranasal aliquot (*e.g*. spray). In some embodiments, each dose is administered as two intranasal aliquots (*e.g*. 2 sprays, one in each nostril.

The aforementioned dosages for the treatment and control of gastroparesis may be administered before meals and/or before bed time. In some embodiments, each dose is administered as a single intranasal aliquot (*e.g*. 1 spray in one nostril); in some embodiments, the dose may be split into 2 or more intranasal aliquots (*e.g*. 2 sprays, one in each nostril).

### EXAMPLES

### Example 1: A Multicenter, Randomized, Double-Blind, Placebo-Controlled, Parallel Group, Dose-Ranging Clinical Study to Evaluate the Efficacy and Safety of Metoclopramide Nasal Spray Solution in Diabetic Subjects with Gastroparesis

The objectives of this study were to evaluate the safety and the effectiveness of two doses of metoclopramide nasal spray solution, 10 mg and 14 mg, compared to placebo in reducing the symptoms of diabetic gastroparesis and to assess the plasma concentrations of two doses of metoclopramide nasal spray in subjects with diabetic gastroparesis following a single dose and at steady state. The aforementioned dosages for the treatment and control of gastroparesis were administered before meals and/or before bed time. Subjects who met the entry criteria after the Washout Period (Day -7 to Day -1) were randomized using an IVRS, to metoclopramide nasal spray 10 mg, 14 mg, or placebo, in roughly equal numbers (approximately 1:1:1) using a predetermined randomization schedule employing permuted blocks. Randomization was preformed centrally.

Metoclopramide nasal spray solution, 200 mg/mL base (as monohydrochloride monohydrate) is a clear colorless to pale-yellow aqueous solution manufactured for Evoke Pharma. The metoclopramide nasal spray was packaged in a 10 mL amber glass vial mounted with a metered nasal spray pump. The metered dose vial delivered a 50 µL or 70 µL spray (10 mg or 14 mg of metoclopramide base respectively) with each actuation. One 10 mL vial contained sufficient metoclopramide nasal spray to deliver 120 doses of 10 mg or 14 mg.

A vehicle control was used as the placebo for metoclopramide nasal spray solution. The placebo spray was packaged in a 10 mL amber glass vial mounted with a metered nasal spray pump. The metered dose vial delivered a 50 µL spray with each actuation. One 10 mL vial contained sufficient placebo to deliver 120 doses. The delivery of either a 50 µL or 70 µL spray is indistinguishable to the subject or study staff.

A 7-day Washout Period preceded randomization. During this time, and for the duration of the study, subjects were asked to discontinue use of all medications that are known to ameliorate or exacerbate symptoms associated with diabetic gastroparesis. Subjects were trained on the use of an interactive voice response system (IVRS) to record the severity of nine gastroparesis symptoms of the GCSI-DD and additional symptoms such as: severity of abdominal pain and abdominal discomfort, number of hours of nausea, number of episodes of vomiting, and overall severity of gastroparesis symptoms in a daily diary. The IVRS was used during the treatment period for self-reported assessments. Subjects completed these diary assessments via the IVRS each evening.

Following the Washout Period, subjects who met all entry criteria and had a mean daily score of ≥2 to ≤4 during the 7-day Washout Period on the nine-symptom GCSI-DD were randomized on Day 0 to one of three treatment groups to receive either metoclopramide nasal spray 10 mg or 14 mg or placebo, one spray in either nostril, four times daily, 30 minutes before meals and at bedtime. Subjects with a mean GCSI-DD total score of <2.0 or >4.0 during the 7-day Washout Period were excluded from the study.

At the Randomization Visit (Day 0), the Patient Assessment of Upper Gastrointestinal Disorders Symptoms (PAGI-SYM) Questionnaire, and disability questionnaires were administered to the subject and the Investigator and subject assessed overall gastroparesis symptom severity (OGS) prior to taking study drug. Subjects began taking the study drug at the clinical site with a single dose on Day 0. The subjects returned to the clinic for safety and efficacy evaluations on Visits 4, 5, 6, and 7 (Days 7, 14, 21, and 28).

At each scheduled study visit to the clinical study site, the IVRS daily diary compliance was reviewed. In addition, at Visit 5 (Day 14), the PAGI-SYM Questionnaire was administered to the subject and the Investigator and subject assessed OGS severity. At the Final Visit (Day 28) subjects took their last dose of study drug at the clinical site and returned their vial of unused/remaining study drug. The subject completed the PAGI-SYM, and disability questionnaires. The Investigator and subject assessed OGS and overall treatment effect (OTE).

Study drug was administered intranasally (one spray in either the right or the left nostril) four times a day, 30 minutes before meals and at bedtime. Subjects were instructed on the correct use of the nasal spray, were reminded not to exceed a total of four sprays a day, and received their first and last dose of study drug in the clinic on Day 0 and Day 28.

Pharmacokinetic (PK) blood samples for the determination of metoclopramide concentration were obtained from all subjects on Visit 3, Day 0 (pre-dose and 30 minutes after dosing [single dose]) and at the Final Visit (Day 28) 30 minutes post-dose (steady state).

The primary efficacy endpoint for this study was the change in mean modified Gastroparesis Cardinal Symptom Index - Daily Diary (mGCSI-DD) total score from the 7-day Baseline (Day -7 to Day -1) to the last 7 days (Day 21 to Day 28 for subjects who complete 28 days of treatment) of the treatment period between each of the two active treatment groups and the placebo group. The mGCSI-DD, a subject-reported assessment of severity of gastroparesis symptoms, will be the instrument used during the washout period (Baseline) and the dosing phase of the study to record daily symptoms. The daily diary scores were recorded using an Interactive Voice Response System (IVRS) at the same time each evening.

The mGCSI-DD was chosen for this study because the instrument was developed specifically to capture the symptoms of gastroparesis and has been tested in subjects with diabetic gastroparesis. The range of mean GCSI-DD scores selected for inclusion in the study was based on the severity of gastroparesis symptoms. Subjects with a mean GCSI-DD score of <2.0 are considered to have very mild symptoms while subjects with a mean GCSI-DD of score >4.0 are considered to have severe to very severe symptoms more appropriately treated by parenteral metoclopramide.

The study consisted of up to a 23-day Screening Period, a 7-day Washout Period (Day - 7 to Day -1) followed by four weeks of study drug treatment. The total duration of each subject's participation was approximately 8 weeks. 287 subjects were enrolled in the study and were randomized to the following groups: Placebo (n=95), 10 mg intranasal (n=96) and 14 mg intranasal (n=96). Of those subjects enrolled, a total of 259 completed the study (∼90%). There were no dose-limiting toxicities and no reports of tardive dyskinesia. Of the study subjects who failed to complete the subject, 4 were from the placebo group, 3 from the 10 mg IN group, and 8 from the 14 mg IN group.

### Symptom Daily Diary

Test subjects were daily asked several questions about their gastroparesis symptoms. The daily diary was completed each evening. For each symptom, subjects were instructed to choose the number (0-5) that best described how severe the symptom has been during the previous 24 hours. Subjects were instructed to answer each question. The following symptom scores were used: 0=None; 1=Very Mild; 2=Mild; 3=Moderate; 4=Severe; 5=Very Severe. The IVRS queried each subject on the following symptoms: 1. Nausea (feeling sick to your stomach as if you were going to vomit or throw up); 2. Retching (heaving as if to vomit, but nothing comes up); 3. Vomiting; 4. Stomach fullness; 5. Not able to finish a normal-sized meal; 6. Feeling excessively full after meals; 7. Loss of appetite; 8. Bloating; 9. Stomach or belly visibly larger; 10. Upper abdominal pain (above the navel); 11. Upper abdominal discomfort (above the navel). The test subjects were asked the following question: "What was the overall severity of your gastroparesis symptoms today (during the past 24 hours)?" "During the past 24 hours, how many episodes of vomiting did you have?" and "During the past 24 hours, how many hours of nausea did you have?"

### Patient Assessment of Upper Gastrointestinal Disorders Symptoms Questionnaire (PAGI-SYM)

This questionnaire asked the subjects about the severity of symptoms related to their gastrointestinal problems. For each symptom, subjects were instructed to select the number that best described how severe the symptom has been during the prior 2 weeks.. If the subject did not experience this symptom, they were to select 0. If the symptom was mild, the subject was instructed to select 1. If it was moderate, the subject was instructed to select 3. If it was severe, the subject was instructed to select 4. If it was very severe, the subject was instructed to select 5. The subjects were instructed to answer each question as accurately as possible, and to answer every question. The symptoms that the subjects rated were: 1. Nausea (feeling sick to your stomach as if you were going to vomit or throw up); 2. Retching (heaving as if to vomit, but nothing comes up); 3. Vomiting; 4. Stomach fullness; 5. Not able to finish a normal-sized meal; 6. Feeling excessively full after meals; 7. Loss of appetite; 8. Bloating (feeling like you need to loosen your clothes); 9. Stomach or belly visibly larger; 10. Upper abdominal pain (above the navel); 11. Upper abdominal discomfort (below the navel); 12. Lower abdominal pain (below the navel); 13. Lower abdominal discomfort (below the navel); 14. Heartburn during the day (burning pain rising in your chest or throat); 15. Heartburn when lying down (burning pain rising in your chest or throat); 16. Feeling of discomfort inside your chest during the day; 17. Feeling of discomfort inside your chest at night (during your sleep time); 18. Regurgitation or reflux during the day (fluid or liquid from your stomach coming up into your throat); 19. Regurgitation or reflux when lying down (fluid or liquid from your stomach coming up into your throat); 20. Bitter, acid, or sour taste in your mouth.

**Primary Efficacy Endpoint** - **ITT:** Change from Baseline to Week 4 in Mean mGCSI-DD. The following table summarizes the mGCSI-DD scores for all subjects (*i.e.,* no gender differentiation) for the Placebo, 10 mg IN and 14 mg IN subject groups (Treatment Arms). As can be seen from the pairwise *p*-values, neither Treatment Arm experienced statistically significant improvement in mGCSI-DD score over Placebo.

| **Time point** | **Placebo** | **10 mg IN** | **14 mg IN** |
|---|---|---|---|
| **Baseline** | 2.8 | 2.9 | 2.9 |
| **Week 4** | 1.8 | 1.6 | 1.7 |
| **Mean Change from Baseline to Week 4** | -1.0 | -1.2 | -1.2 |
| **Pairwise *p*-value v. placebo** | N/A | 0.1504 | 0.3005 |
| **Pairwise *p*-value v. 10 mg** | N/A | N/A | 0.6830 |

**Primary Efficacy Endpoint - ITT - By Gender**: Changes from Baseline to Week 4 in Mean mGCSI-DD for Females (Males). When subjects were classified by gender (as prespecified in the statistical analysis plan), there was a clear difference in mGCSI-DD scores between females and males. As can be seen in the *p*-values in the following table, the females in both the 10 mg IN and 14 mg IN groups (Treatment Arms) experienced statistically significant improvement in mGCSI-DD scores versus placebo, whereas males in both Treatment Arms did not.

| **Time point** | **Placebo** | **10 mg IN** | **14 mg IN** |
|---|---|---|---|
| Baseline | 2.7 (2.9) | 2.9 (2.8) | 2.9 (2.5) |
| Week 4 | 1.9 (1.4) | 1.6 (1.6) | 1.7 (1.7) |
| Mean Change from Baseline to Week 4 | -0.8 (-1.4) | -1.2 (-1.2) | -1.3 (-0.9) |
| Pairwise *p*-value v. placebo | N/A | **0.0247** (0.4497) | **0.0215** (0.2174) |
| Pairwise *p*-value v. 10 mg | N/A | N/A | 0.69864 (0.5805) |

**Primary and Secondary Endpoints by Gender at Week 4.** The following table summarizes the results (A = Favors Active, P = Favors Placebo) for the primary endpoint (mGCSI-DD score) and secondary endpoints of the study. As can be seen in the table, in females of both Treatment Arms (10 mg and 14 mg), the results of the mGCSI-DD score and the various secondary endpoints favor active (metoclopramide intranasal). In contrast, in males of both Treatment Arms, the mGCSI-DD scores favored placebo, as did every secondary endpoint other than vomiting.

| | **Females** | | **Males** | |
|---|---|---|---|---|
| | **10 mg** | **14 mg** | **10 mg** | **14 mg** |
| **Primary Endpoints** | | | | |
| mGCSI-DD | A (*p*<0.05) | A (*p*<0.05) | P | P |
| **Secondary Endpoints** | | | | |
| Nausea | A (*p*<0.05) | A (*p*<0.05) | P | P |
| Retching | A | A | P | P |
| Vomiting | A | A | A | A |
| Stomach Fullness | A | A | P | P |
| Early Satiety | A | A | P | P (*p*<0.05) |
| Feeling Full | A (*p*<0.05) | A | P | P |
| Loss of Appetite | A | A | P | P (*p*<0.05) |
| Bloating | A | A | P | P |
| Stomach Larger | A | A (*p*<0.05) | P | P (*p*<0.05) |
| Upper Abdominal Pain | A (*p*<0.05) | A (*p*<0.05) | P | P |
| Upper Abdominal Discomfort | A | A | P | P |

Exploratory Endpoints by Gender at Week 4. Various exploratory endpoints were also followed by the study. The following table summarizes the results (A = Favors Active, P = Favors Placebo) for the primary endpoint (mGCSI-DD score) and exploratory endpoints of the study. As can be seen in the table, in females of both Treatment Arms (10 mg and 14 mg) favor active (metoclopramide intranasal) according the GCSI-DD score. Likewise, according females favored active for the majority of exploratory endpoints. In contrast, in males of both Treatment Arms, the GCSI-DD scores favored placebo, as did most of the exploratory endpoints.

| | **Females** | | **Males** | |
|---|---|---|---|---|
| **Exploratory Endpoints** | **10 mg** | **14 mg** | **10 mg** | **14 mg** |
| GCSI-DD | A (*p*<0.05) | A (*p*<0.05) | P | P |
| Days w/o Vomiting or Retching & No Use of Rescue Medication | A | A | A | A |
| Nausea Responder Analysis | A | A (*p*<0.05) | P | P |
| Overall Severity of Gastroparesis Symptoms - Subject | A (*p*<0.05) | A | A | A |
| PAGI-SYM Questionnaire | A | A | P | P |
| Sheehan Disability Scales | A | A | A | A |
| Investigator Assessment of Overall Gastroparesis Severity | A | A | P | P |
| Subject Assessment of Overall Gastroparesis Severity | A | A | P | P |
| Investigator Assessment of Overall Treatment Effect | P | A | A | P |
| Subject Assessment of Overall Treatment Effect | A | A | A | P |
| Days w/o Vomiting | A | A | P | P |
| Days w/o Nausea | A (*p*<0.05) | A (*p<*0.05) | P | P |
| Days w/o Vomiting or Nausea | A (*p*<0.05) | A (*p*<0.05) | P | P |
| Days w/o Upper Abdominal Pain or Upper Abdominal Discomfort | A | P | P | P (p<0.05) |
| Nausea Response by Response Threshold | A (*p*<0.05) | A (*p*<0.05) | P | P |

Subject Demographics - ITT: The following table shows that the dose groups are comparable across multiple characteristics, including Baseline Mean GCSI-DD and mGCSI-DD

METO-IN-002: Demographics and Baseline Characteristics (ITT Population)

| | **Placebo (N = 95)** | **Metoclopramide 10 mg IN (N = 96)** | **Metoclopramide 14 mg IN (N = 96)** |
|---|---|---|---|
| **Age (years)** | | | |
| Mean (SD) | 52.4 (10.03) | 51.6 (12.08) | 50.4 (12.40) |
| **Sex** | | | |
| Male | 27 (28.4%) | 31 (32.3%) | 26 (27.1%) |
| Female | 68 (71.6%) | 65 (67.7%) | 70 (72.9%) |
| **Ethnicity** | | | |
| Hispanic or Latino | 19 (20.0%) | 7 (7.3%) | 13 (13.5%) |
| Not Hispanic or Latino | 76 (80.0%) | 89 (92.7%) | 83 (86.5%) |
| **Race** | | | |
| Asian | 0 (0.0%) | 1 (1.0%) | 0 (0.0%) |
| Black or African American | 23 (24.2%) | 28 (29.2%) | 31 (32.3%) |
| White | 67 (70.5%) | 62 (64.6%) | 65 (67.7%) |
| Other | 3 (3.2%) | 2 (2.1%) | 0 (0.0%) |
| Multiple Races Checked | 2 (2.1%) | 3 (3.1%) | 0 (0.0%) |
| **BMI (kg/m²)** | | | |
| Mean (SD) | 34.6 (7.96) | 33.5 (8.01) | 32.9 (7.89) |
| **GCSI-DD** | | | |
| Mean (SD) | 2.7 (0.49) | 2.7 (0.49) | 2.7 (0.47) |
| **mGCSI-DD** | | | |
| Mean (SD) | 2.8 (0.57) | 2.9 (0.60) | 2.8 (0.62) |
| **Diabetes Mellitus Type** | | | |
| Type 1 | 15 (15.8%) | 17 (17.7%) | 19 (19.8%) |
| Type 2 | 80 (84.2%) | 79 (82.3%) | 77 (80.2%) |

Subject Demographics - ITT by Gender (M/F): The following table shows that there were no gender-based differences in demographics or symptom scores at baseline.

METO-IN-002: Key Demographics and Baseline Characteristics by Gender (ITT Population)

| **Parameter** | **Placebo** | | **Metoclopramide 10 mg IN** | | **Metoclopramide 14 mg IN** | |
|---|---|---|---|---|---|---|
| | **Male (N=27)** | **Female (N=68)** | **Male (N=31)** | **Female (N=65)** | **Male (N=26)** | **Female (N=70)** |
| **Age (years)** | | | | | | |
| Mean | 51.2 | 52.9 | 51.3 | 51.8 | 55.0 | 48.7 |
| **BMI (kg/m²)** | | | | | | |
| Mean | 34.7 | 34.6 | 32.3 | 34.0 | 31.4 | 33.5 |
| **GCSI-DD** | | | | | | |
| Mean | 2.8 | 2.7 | 2.7 | 2.7 | 2.6 | 2.8 |
| **mGCSI-DD** | | | | | | |
| Mean | 2.9 | 2.7 | 2.8 | 2.9 | 2.5 | 2.9 |
| **Diabetes Mellitus** | | | | | | |
| **Type** | | | | | | |
| Type 1 | 6 (22%) | 9 (13%) | 4 (13%) | 13 (20%) | 4 (15%) | 15 (21%) |
| Type 2 | 21 (78%) | 59 (87%) | 27 (87%) | 52 (80%) | 22 (85%) | 55 (79%) |

Statistical analysis of top line data interaction test demonstrated the interaction between treatment, gender and the primary endpoint - mGCSI-DD change from Baseline to Week 4 is significantly different between males compared to females (*p* = 0.0381). As can be seen in the following table, treatment effects appear opposite in males and females.

| **Time Point** | **Placebo (N=95)** | **Metoclopramide 10 mg IN (N=96)** | **Metoclopramide 14 mg IN (N=96)** |
|---|---|---|---|
| **ALL SUBJECTS** | | | |
| Baseline^{[1]} | | | |
| N | 95 | 96 | 96 |
| Mean (SD) | 2.8 (0.57) | 2.9 (0.60) | 2.8 (0.62) |
| Week 4 | | | |
| N | 95 | 96 | 96 |
| Mean (SD) | 1.8 (1.00) | 1.6 (1.06) | 1.7 (0.90) |
| Change from Baseline to Week 4 | | | |
| N | 95 | 96 | 96 |
| Mean (SD) | -1.0 (0.89) | -1.2 (1.18) | -1.2 (0.94) |
| Difference of Least Square Means (95% CI) | | -0.20 (-0.47, 0.07) | -0.14 (-0.42, 0.13) |
| Pairwise p-value vs. Placebo^{[2]} | | 0.1504 | 0.3005 |
| Difference of Least Square Means (95% CI) | | | 0.06 (-0.22, 0.33) |
| Pairwise p-value vs. Metoclopramide 10 mg^{[2]} | | | 0.6830 |
| P-value for Treatment by Gender Interaction ^{[3]} | **0.0381** | | |

| **FEMALES** | | | |
|---|---|---|---|
| Baseline^{[1]} | | | |
| N | 68 | 65 | 70 |
| Mean (SD) | 2.7 (0.54) | 2.9 (0.62) | 2.9 (0.62) |
| Week 4 | | | |
| N | 68 | 65 | 70 |
| Mean (SD) | 1.9 (1.02) | 1.6 (1.08) | 1.7 (0.94) |
| Change from Baseline to Week 4 | | | |
| N | 68 | 65 | 70 |
| Mean (SD) | -0.8 (0.79) | -1.2 (1.18) | -1.3 (0.98) |
| Difference of Least Square Means (95% CI) | | -0.38 (-0.71, -0.05) | -0.38 (-0.71, -0.06) |
| Pairwise p-value vs. Placebo^{[2]} | | **0.0247** | **0.0215** |
| Difference of Least Square Means (95% CI) | | | -0.00 (-0.33, 0.32) |
| Pairwise p-value vs. Metoclopramide 10 mg^{[2]} | | | 0.9864 |

| **MALES** | | | |
|---|---|---|---|
| Baseline^{[1]} | | | |
| N | 27 | 31 | 26 |
| Mean (SD) | 2.9 (0.63) | 2.8 (0.54) | 2.5 (0.56) |
| Week 4 | | | |
| N | 27 | 31 | 26 |
| Mean (SD) | 1.4 (0.84) | 1.6 (1.05) | 1.7 (0.79) |
| Change from Baseline to Week 4 | | | |
| N | 27 | 31 | 26 |
| Mean (SD) | -1.4 (0.98) | -1.2 (1.21) | -0.9 (0.78) |
| Difference of Least Square Means (95% CI) | | 0.18 (-0.30, 0.66) | 0.32 (-0.19, 0.83) |
| Pairwise p-value vs. Placebo^{[2]} | | 0.4497 | 0.2174 |
| Difference of Least Square Means (95% CI) | | | 0.14 (-0.35, 0.63) |
| Pairwise p-value vs. Metoclopramide 10 mg^{[2]} | | | 0.5805 |

| | | | |
|---|---|---|---|
| ^{[1]} Baseline is defined as the mean mGCSI-DD total score during the Washout Period ^{[2]} P-values for pairwise comparisons are obtained from an ANCOVA model with effects for treatment group and Baseline value as a covariate ^{[3]} P-value for gender by treatment interaction test is obtained from an ANCOVA model with effects for treatment group, gender, treatment by gender interaction, and Baseline value as a covariate | | | |

Results for key mGCSI-DD Symptoms: Nausea, bloating, early satiety and abdominal pain

| | **Females** | | **Males** | |
|---|---|---|---|---|
| **Endpoints** | **10 mg** | **14 mg** | **10 mg** | **14 mg** |
| **mGCSI-DD** | A (*p*<0.05) | A (*p*<0.05) | P | P |
| Nausea | A (*p*<0.05) | A (*p*<0.05) | P | P |
| Bloating | A | A | P | P |
| Early Satiety | A | A | P | P |
| Upper Abdominal Pain | A (*p*<0.05) | A (*p*<0.05) | P | P |
| **GCSI-DD** | A (*p*<0.05) | A (*p*<0.05) | P | P |
| Feeling Full | A (*p*<0.05) | A | P | P |
| Stomach Larger | A | A (*p*<0.05) | P | P (*p*<0.05) |
| **PAGI-SYM Questionnaire** | A | A | P | P |
| Nausea/Vomiting | A | A (*p*<0.05) | P | P |
| Bloating | A | A | P | P (*p*<0.05) |
| Upper Abdominal Pain/Discomfort | A | A | P | P |
| Early Satiety | A | A | P | P (*p*<0.05) |
| **Days w/o Nausea** | A (*p*<0.05) | A (*p*<0.05) | P | P |
| **Nausea Responder Analysis** | A | A (*p*<0.05) | P | P |
| **Nausea Response by Response Threshold** | A (*p*<0.05) | A (*p*<0.05) | P | P |
| **Days w/o Vomiting or Nausea** | A (*p*<0.05) | A (*p*<0.05) | P | P |
| **Days w/o Upper Abdominal Pain or Upper Abdominal Discomfort** | A | P | P | P (*p*<0.05) |

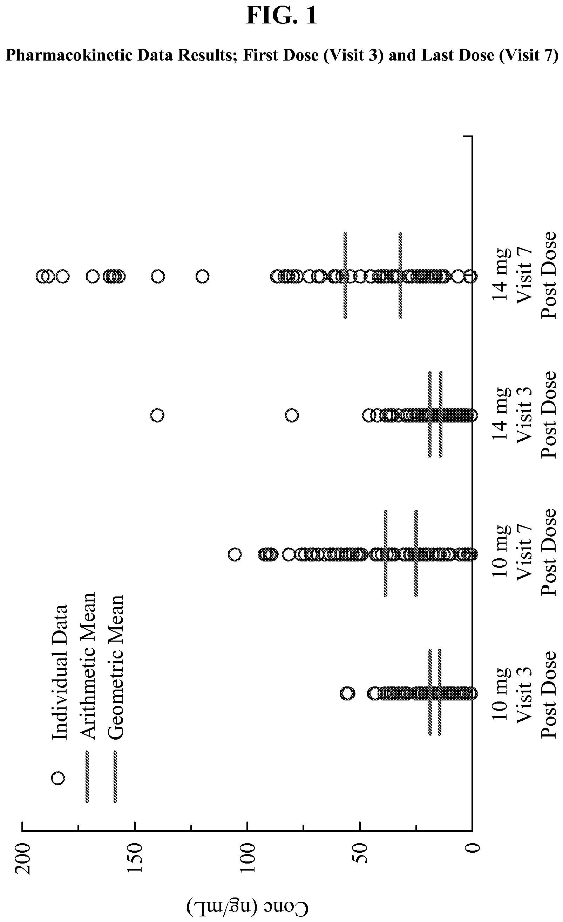

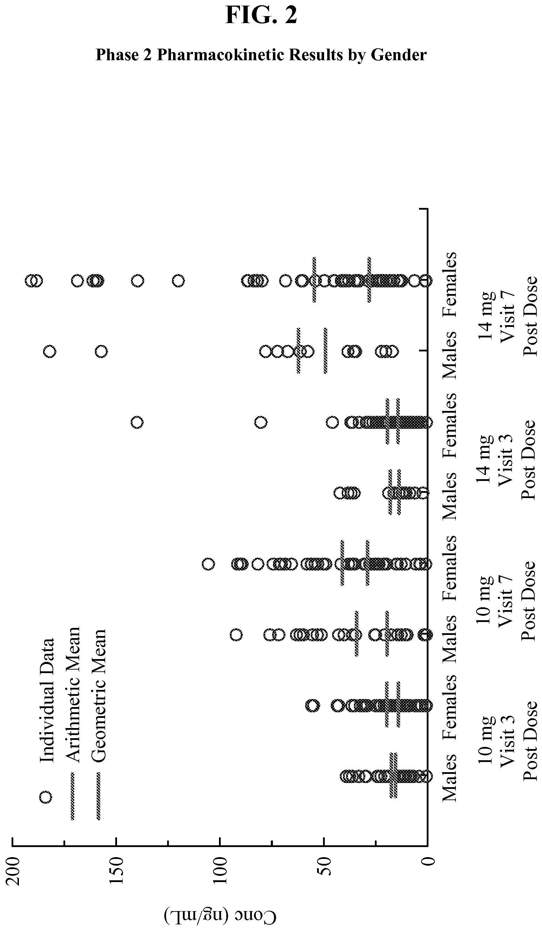

Pharmacokinetics: The pharmacokinetics (PK) of the study drug was evaluated in all subjects. Figure 1 summarizes PK data (blood plasma concentration of metoclopramide in ng/mL) for both Treatment Arms (10 mg and 14 mg, intranasal) on visit 3 and visit 7. Individual data are represented by circles and the arithmetic mean and geometric mean of each group is represented by lines. (In each case, the upper line represents the arithmetic mean and the lower line represents the geometric mean.) In Figure 2, the PK data are analyzed by gender. As in Figure 1, the circles represent individual data, whereas the lines represent means (upper line = arithmetic mean; lower line = geometric mean.) As can be seen in Figure 2, the PK data for males and females were similar, when compared at like dosages on the same days. Statistical analysis revealed that any apparent differences in mean values between females and males were not statistically significant.

Phase 2 Summary: As can be seen in the tables above, for female subjects, 11 of 11 symptoms for which data were collected had statistical separation or improved trends compared to placebo (p = 0.001). In contrast, male subjects had only 1 of 11 symptoms that showed a positive trend (p = 0.0118). These differences cannot be rationalized based upon differences in pharmacokinetics between females and males, as no statistical difference in PK values was seen between females and males in either Treatment Arm (10 mg, 14 mg) on either of the days (Visit 3, Visit 7) tested. The similarity in PK data between the two gender groups suggests that it is unlikely that the difference in efficacy can be explained as a difference in relative dosing. Thus it is not clear from these results that increasing the dose in males would enhance efficacy in that group. There were no apparent demographic differences between females and males that would explain the difference in efficacy between the two groups. From these results, it is reasonable to infer that intranasal metoclopramide is effective in treating female gastroparesis but not male gastroparesis. At the least, it is rational to conclude that at the doses studied - 10 mg and 14 mg - intranasal metoclopramide is effective for the treatment of symptoms associated with female diabetic gastroparesis, but not in the treatment of symptoms associated with male diabetic gastroparesis.

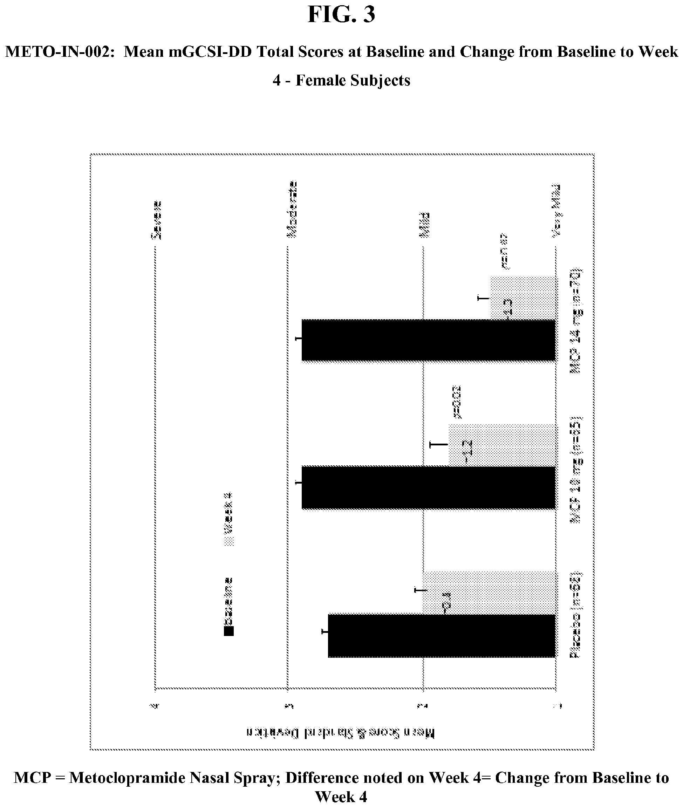

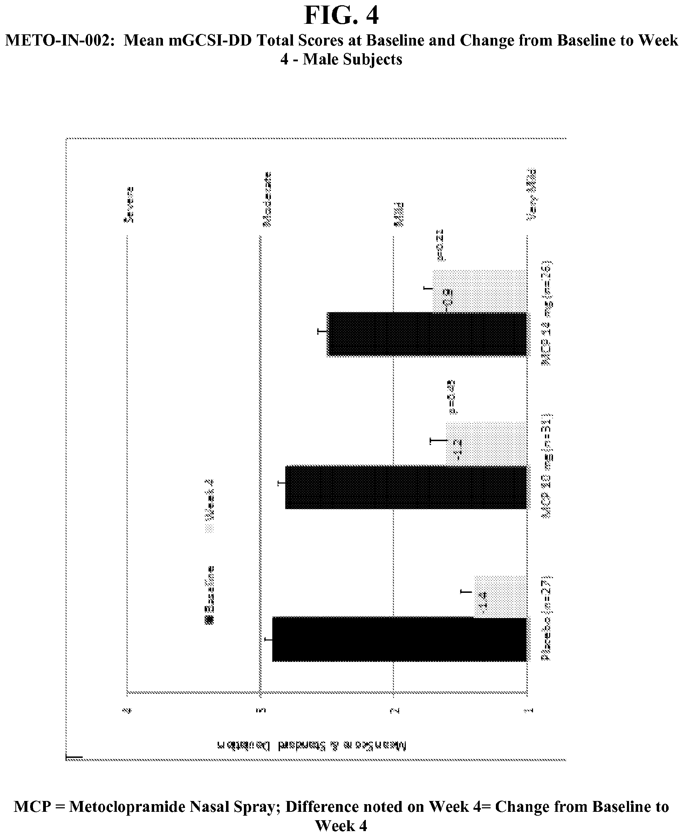

Figures 3 and 4 graphically depict the mean mGCSI-DD total scores at baseline and change from baseline to week 4 in female and male subjects, respectively. As can be seen in these graphs, female subjects experienced statistically significant improvement in mGCSI-DD from baseline at both 10 mg and 14 mg doses (corresponding to daily doses of 40 mg and 56 mg, respectively), whereas male subjects did not experience significant improvement.

## Claims

1. A formulation of metoclopramide for intranasal administration comprising metoclopramide, citrate buffer, and benzalkonium chloride, wherein the formulation has a pH of at least 5 and wherein the composition comprises 5mg to 25mg of metoclopramide per aliquot, for use in the treatment of one or more symptoms associated with diabetic gastroparesis in human females, and is for administration to diabetic human females in a daily dose of 3-8 intranasal aliquots.

2. The composition for use of any one of claim 1, wherein the composition of metoclopramide is for administration at a daily dose of 20 mg to 100 mg of metoclopramide base per day.

3. The composition for use of any one of claim 2, wherein the composition of metoclopramide is for administration at a daily dose of 40 mg to 80 mg of metoclopramide base per day.

4. The composition for use of any one of claims 1-3, wherein the daily dose of the composition of metoclopramide is for administration as aliquots having a volume of 25 µL to 150 µL.

5. The composition for use of any one of claims 1-4, wherein the metoclopramide, or pharmaceutically acceptable salt thereof, is administered in a 50 microliter aliquot, four times per day.

6. The composition for use of any one of claims 1-4, wherein the metoclopramide, or pharmaceutically acceptable salt thereof, is administered in a 70 microliter aliquot, four times per day.

7. The composition for use of any one of claims 1-6, wherein the one or more symptoms of diabetic gastroparesis in human females includes one or more symptoms selected from the group consisting of nausea, retching, vomiting, stomach fullness, loss of appetite, bloating, upper abdominal pain, and upper abdominal discomfort.

8. The composition for use of any one of claims 1-7, for treatment of two or more symptoms of diabetic gastroparesis in human females.

9. The composition for use of claims 1-8, wherein the two or more symptoms of diabetic gastroparesis in human females include upper abdominal pain, upper abdominal discomfort, vomiting, and nausea.

## Patentansprüche

1. Formulierung von Metoclopramid zur intranasalen Verabreichung, umfassend Metoclopramid, Citratpuffer und Benzalkoniumchlorid, wobei die Formulierung einen pH von mindestens 5 aufweist und wobei die Zusammensetzung 5mg bis 25mg Metoclopramid pro Aliquot umfasst, zur Verwendung in der Behandlung von einem oder mehreren mit diabetischer Gastroparese in weiblichen Menschen assoziierten Symptomen, und die zur Verabreichung an diabetische weibliche Menschen in einer täglichen Dosis von 3-8 intranasalen Aliquots ist.

2. Zusammensetzung zur Verwendung nach einem beliebigen von Anspruch 1, wobei die Zusammensetzung von Metoclopramid zur Verabreichung bei einer täglichen Dosis von 20 mg bis 100 mg Metoclopramidbase pro Tag ist.

3. Zusammensetzung zur Verwendung nach einem beliebigen von Anspruch 2, wobei die Zusammensetzung von Metoclopramid zur Verabreichung bei einer täglichen Dosis von 40 mg bis 80 mg Metoclopramidbase pro Tag ist.

4. Zusammensetzung zur Verwendung nach einem beliebigen der Ansprüche 1-3, wobei die tägliche Dosis der Zusammensetzung von Metoclopramid zur Verabreichung als Aliquots mit einem Volumen von 25 µL bis 150 µL ist.

5. Zusammensetzung zur Verwendung nach einem beliebigen der Ansprüche 1-4, wobei das Metoclopramid oder das pharmazeutisch verträgliche Salz davon in einem 50 Mikroliteraliquot viermal pro Tag verabreicht wird.

6. Zusammensetzung zur Verwendung nach einem beliebigen der Ansprüche 1-4, wobei das Metoclopramid oder das pharmazeutisch verträgliche Salz davon in einem 70 Mikroliteraliquot viermal pro Tag verabreicht wird.

7. Zusammensetzung zur Verwendung nach einem beliebigen der Ansprüche 1-6, wobei das eine oder die mehreren Symptome von diabetischer Gastroparese in weiblichen Menschen ein oder mehrere Symptome einschließt, ausgewählt aus der Gruppe bestehend aus Übelkeit, Würgen, Erbrechen, Völlegefühl, Appetitverlust, Blähungen, Oberbauchschmerzen und Oberbauchbeschwerden.

8. Zusammensetzung zur Verwendung nach einem beliebigen der Ansprüche 1-7, zur Behandlung von zwei oder mehr Symptomen von diabetischer Gastroparese in weiblichen Menschen.

9. Zusammensetzung zur Verwendung nach Ansprüchen 1-8, wobei die zwei oder mehr Symptome von diabetischer Gastroparese in weiblichen Menschen Oberbauchschmerzen, Oberbauchbeschwerden, Erbrechen und Übelkeit einschließen.

## Revendications

1. Formulation de métoclopramide pour une administration intranasale comprenant du métoclopramide, du tampon citrate et du chlorure de benzalkonium, dans laquelle la formulation a un pH d'au moins 5 et dans laquelle la composition comprend 5 mg à 25 mg de métoclopramide par aliquote, pour une utilisation dans le traitement d'un ou de plusieurs symptômes associés à la gastroparésie diabétique chez la femme, et est destinée à l'administration à des femmes diabétiques à une dose quotidienne de 3 à 8 aliquotes intranasales.

2. Composition pour une utilisation selon l'une quelconque de la revendication 1, dans laquelle la composition de métoclopramide est destinée à l'administration à une dose quotidienne de 20 mg à 100 mg de métoclopramide base par jour.

3. Composition pour une utilisation selon l'une quelconque de la revendication 2, dans laquelle la composition de métoclopramide est destinée à l'administration à une dose quotidienne de 40 mg à 80 mg de métoclopramide base par jour.

4. Composition pour une utilisation selon l'une quelconque des revendications 1 à 3, dans laquelle la dose quotidienne de la composition de métoclopramide est destinée à l'administration sous la forme d'aliquotes ayant un volume de 25 µl à 150 µl.

5. Composition pour une utilisation selon l'une quelconque des revendications 1 à 4, dans laquelle le métoclopramide, ou un sel pharmaceutiquement acceptable de celui-ci, est administré dans une aliquote de 50 microlitres, quatre fois par jour.

6. Composition pour une utilisation selon l'une quelconque des revendications 1 à 4, dans laquelle le métoclopramide, ou un sel pharmaceutiquement acceptable de celui-ci, est administré dans une aliquote de 70 microlitres, quatre fois par jour.

7. Composition pour une utilisation selon l'une quelconque des revendications 1 à 6, dans laquelle le ou les symptômes de gastroparésie diabétique chez la femme comprennent un ou plusieurs symptômes choisis dans le groupe constitué par des nausées, un haut-le-coeur, des vomissements, une sensation d'estomac plein, une perte d'appétit, des ballonnements, une douleur à l'épigastre, et une gêne dans la partie supérieure de l'abdomen.

8. Composition pour une utilisation selon l'une quelconque des revendications 1 à 7, pour le traitement de deux symptômes de gastroparésie diabétique ou plus chez la femme.

9. Composition pour une utilisation selon l'une quelconque des revendications 1 à 8, dans laquelle les deux symptômes de gastroparésie diabétique ou plus chez la femme comprennent une douleur à l'épigastre, une gêne dans la partie supérieure de l'abdomen, des vomissements, et des nausées.
